# EUROPEAN PATENT APPLICATION

(11) **EP 3 245 975 A1**
(43) Date of publication of application: **22.11.2017**
(21) Application number: 15878041.1
(22) Date of filing: 24.12.2015
(51) Int. Cl.: A61B 90/00, B25J 13/02

(54) **OPERATION INPUT DEVICE AND MEDICAL MANIPULATOR SYSTEM**

(30) Priority: 16.01.2015 JP 2015006864
(71) Applicant: Olympus Corporation, Hachioji-shi, Tokyo 192-8507 (JP)
(72) Inventor: OGAWA, Ryohei, Hachioji-shi, Tokyo 192-8507 (JP); KISHI, Kosuke, Hachioji-shi, Tokyo 192-8507 (JP)
(74) Representative: Schicker, Silvia
(86) International application number: PCT/JP2015/086137
(87) International publication number: WO 2016/114090

(57) **Abstract**

The main function of an end effector is manipulated while keeping the orientation of the end effector stable, and limitations in the manipulation amount due to the movable range of the wrist are alleviated. Provided is an operation input device (2) for inputting a manipulation command to a manipulator having an end effector for observing or medically treating an affected site. The operation input device (2) includes a grip section (25) that is holdable by changing a method of holding the grip section with the same hand of an operator (0) and an operating section (16) that is provided in the grip section (25) and that is operated with a single finger, which is the same before and after the holding method is changed, so as to manipulate a main function of the end effector.

## Description

### {Technical Field}

The present invention relates to operation input devices and medical manipulator systems.

### {Background Art}

In a known master-and-slave-type medical manipulator system in the related art, an operation input device operated by an operator is used by the operator using two fingers to open and close a pair of gripping components provided in an operating section (for example, see Patent Literature 1). In this operation input device, a gripper, which is an end effector serving as a slave-side treatment tool, is opened and closed by opening and closing the gripping components.

Another known operation input device includes a pen-type operating section. In this device, the gripper is opened and closed by means of a lever provided in the operating section, and the linkage between the master side and the slave side is turned on and off by means of a switch provided on a side surface of the operating section (for example, see Patent Literature 2).

### Literature 2).

Furthermore, there is another known operation input device used by attaching an operating section having a structure analogous to that of a slave-side treatment tool to the operator's hand and allowing the operator to hold the distal end like a pen (for example, see Patent Literature 3). In this operation input device, the amount by which the gripper is opened and closed is controlled in accordance with the magnitude of force detected by a pressure-sensitive sensor provided at the distal end.

### {Citation List}

### {Patent Literature}

{PTL 1} U.S. Patent No. 8,638,057
{PTL 2} Japanese Translation of PCT International Application, Publication No. 2010-525838

### {Summary of Invention}

### {Technical Problem}

However, because the gripper is opened and closed by using two fingers to operate the gripping components provided in the operating section in the operation input device according to Patent Literature 1, there are problems in that the operating section cannot be held stably with the three remaining fingers, and in that a reactive force of the force applied to the gripping components from the two fingers causes the position of the operating section to fluctuate.

Meanwhile, the operation input device according to Patent Literature 2 is of a type in which the pen-type operating section is held like a pen and the method of holding it is limited. This is problematic in that the manipulation amount is limited due to the small movable range of the wrist of the hand holding the operating section.

The present invention has been made in view of the circumstances described above, and an object thereof is to provide an operation input device and a medical manipulator system with which the main function of an end effector can be manipulated while keeping the orientation of the end effector stable, and with which limitations in the manipulation amount due to the movable range of the wrist can be alleviated.

### {Solution to Problem}

In order to achieve the aforementioned object, the present invention provides the following solutions.

A first aspect of the present invention provides an operation input device for inputting a manipulation command to a manipulator having an end effector for observing or medically treating an affected site. The operation input device includes a grip section that is holdable by changing a method of holding the grip section with the same hand of an operator, and also includes an operating section that is provided in the grip section and that is operated with a single finger, which is the same before and after the holding method is changed, so as to manipulate a main function of the end effector.

In the operation input device according to this aspect, the operator holds the grip section and operates the operating section provided in the grip section, so that a manipulation command for manipulating the main function of the end effector provided in the manipulator is input, whereby the patient is observed or medically treated. Because the operating section is operated with a single finger of the operator, the operating section can be operated in a stable state where the grip section is being firmly held with the remaining fingers excluding the single finger, so that the main function of the end effector can be manipulated while keeping the orientation of the end effector stable.

Furthermore, from a state where the operator is moving the end effector by moving the grip section of the operating section held with one hand and is manipulating the main function of the end effector by operating the operating section with a single finger of that hand, the operator can change the method of holding the grip section with the same hand holding the grip section. Accordingly, even in a case where the movable range of the wrist is limited in accordance with how the grip section is held, the movable range can be expanded by changing the holding method, thereby alleviating the problem of the limited manipulation amount. When the method of holding the grip section is changed, the finger operating the operating section does not change before and after the holding-method changing operation, so that the operator can continue to operate the operating section without being confused.

The operation input device according to the above aspect may further include a sensor that is provided in the grip section and that detects a holding state of a hand of the operator holding the grip section.

Accordingly, when the sensor detects that the grip section is held by the operator's hand, the operating section may be activated or the manipulator may be made manipulatable using the operation input device.

In the operation input device according to the above aspect, the sensor may be a contact sensor.

Accordingly, even when the operator is holding the grip section lightly, the held state can be detected so long as the hand holding the grip section is in contact with the contact sensor, thereby reducing the work required from the operator.

In the operation input device according to the above aspect, the sensor may detect a holding-method changing operation performed on the grip section by the operator.

According to this configuration, when the sensor detects a holding-method changing operation performed on the grip section by the operator, the operation input device and the manipulator are continuously connected to each other without being disconnected, so that smooth medical treatment can be performed without interruption. In contrast, the operation input device and the manipulator are disconnected from each other when the sensor detects a holding-method changing operation performed on the grip section by the operator, thereby preventing unintended movement of the manipulator caused by unintended movement of the grip section during the holding-method changing operation.

In the operation input device according to the above aspect, the operating section may be a slide sensor that adjusts an amount by which the main function of the end effector is manipulated when the single finger is slid relative to the grip section.

Accordingly, by operating the slide sensor by sliding the single finger, the amount by which the main function of the end effector is manipulated can be adjusted without applying force that causes the orientation of the grip section to fluctuate. In other words, the orientation of the grip section does not fluctuate even when the operating section is operated, so that the end effector can be manipulated in a state where the orientation thereof is made more stable.

The operation input device according to the above aspect may further include a main device unit and an arm unit that connects the grip section to the main device unit in a movable manner, and the arm unit may include a rotational supporter that supports the grip section in a rotatable manner about a longitudinal axis thereof.

Accordingly, in a case where the operator moves the grip section while manually holding it, the position and orientation of the grip section change relative to the main device unit. The arm unit deforms so as to follow the change, and the manipulator is moved based on the amount of deformation. When operating the operating section with the single finger of the hand holding the grip section, if the arm unit is to be deformed in conformity to fluctuations in the orientation of the grip section, the grip section rotates about its longitudinal axis depending on the shaft configuration of the arm unit. Thus, the rotation of the grip section is cancelled by rotation of the rotational supporter, so that the grip section can be held comfortably.

A second aspect of the present invention provides a medical manipulator system including: a manipulator having an end effector for observing or medically treating an affected site; the aforementioned operation input device; and a controller that controls the manipulator based on a manipulation command input via the operation input device.

In the medical manipulator system according to this aspect, the operator inputs a manipulation command via the operation input device, so that the controller controls the manipulator based on the manipulation command, and the affected site is observed or medically treated by means of the manipulator. In this case, in the operation input device, the method in which the grip section is held by the operator is changeable, and the main function of the end effector can be manipulated with the same finger before and after the holding-method changing operation. Therefore, the main function of the end effector can be manipulated while keeping the orientation of the end effector stable, and limitations in the manipulation amount due to the movable range of the wrist can be alleviated.

In the medical manipulator system according to the above aspect, the operation input device may include a position detector that detects a position of the hand of the operator relative to the grip section, and the controller may change a command signal for the manipulator in accordance with the position of the hand of the operator detected by the position detector.

According to this configuration, when the position detector detects the position of the operator's hand relative to the grip section, the controller changes the command signal for the manipulator in accordance with the detected position of the hand, so that the manipulator can be smoothly manipulated regardless of whether the method of holding the grip section is changed.

In the medical manipulator system according to the above aspect, the operation input device may include a plurality of the operating sections, and the controller may change a correspondence relationship between each operating section and the main function of the end effector in accordance with the position of the hand of the operator detected by the position detector.

This configuration enables improved ease of operation using the operating section disposed at a position suitable for each holding method in accordance with how the grip section is held.

The medical manipulator system according to the above aspect may further include a type detector that detects a type of the manipulator, and the controller may change a correspondence relationship between the operating section and the main function of the end effector in accordance with the type of the manipulator detected by the type detector.

According to this configuration, the controller changes the correspondence relationship between each operating section and the main function of the end effector in accordance with the type of manipulator detected by the type detector, so that the manipulator can be manipulated using the same operation input device even if the manipulator is replaced with another one.

A third aspect of the present invention provides a medical manipulator system including: a manipulator having an end effector for observing or medically treating an affected site; the aforementioned operation input device; and a controller that controls the manipulator based on a manipulation command input via the operation input device. In a case where the sensor detects that the grip section is not held by the hand of the operator, the controller performs control so as to stop the manipulator from moving.

In the medical manipulator system according to this aspect, when the sensor detects that the grip section is not held by the operator, the manipulator is stopped, thereby preventing unintended movement of the manipulator due to unintended movement of the grip section caused when the grip section is released during a holding-method changing operation.

### {Advantageous Effects of Invention}

The present invention is advantageous in that the main function of an end effector can be manipulated while keeping the orientation of the end effector stable, and limitations in the manipulation amount due to the movable range of the wrist can be alleviated.

### {Brief Description of Drawings}

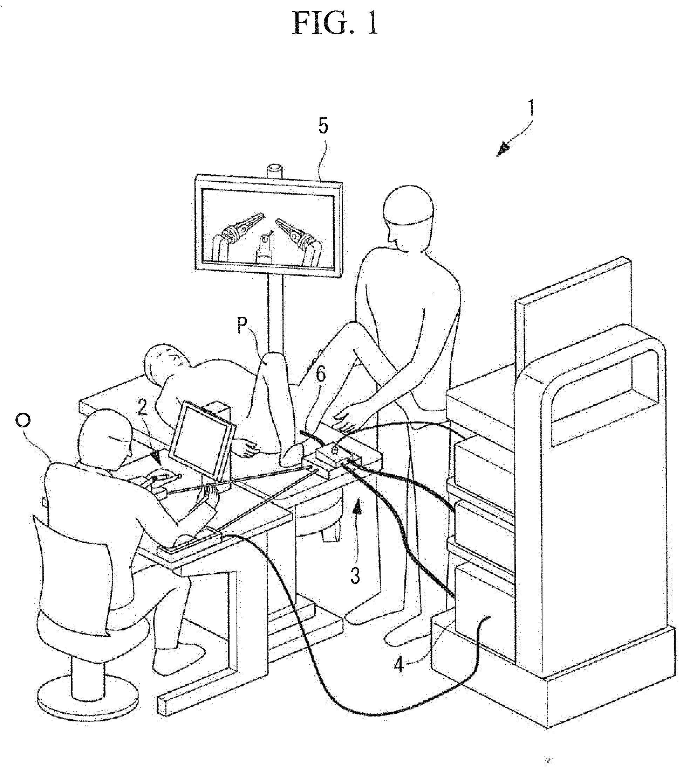

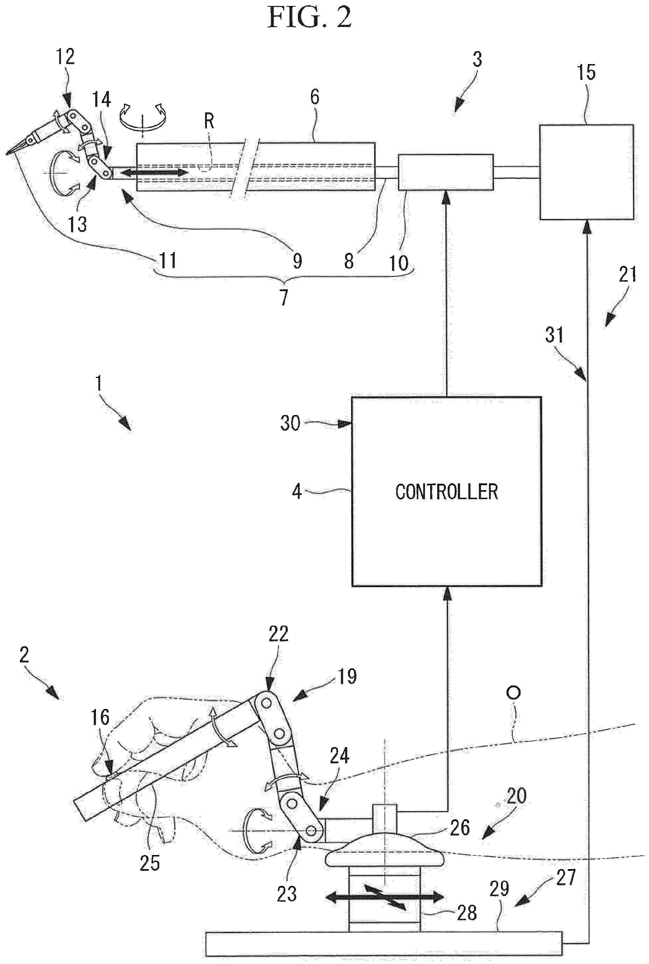

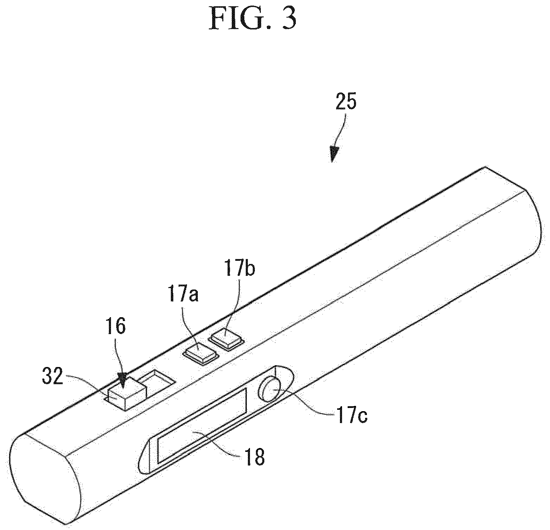

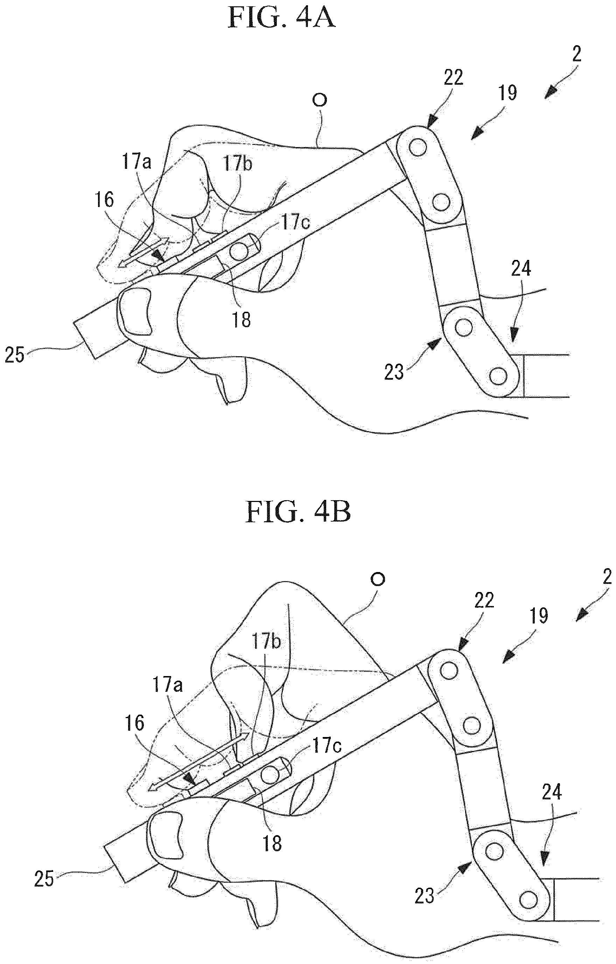

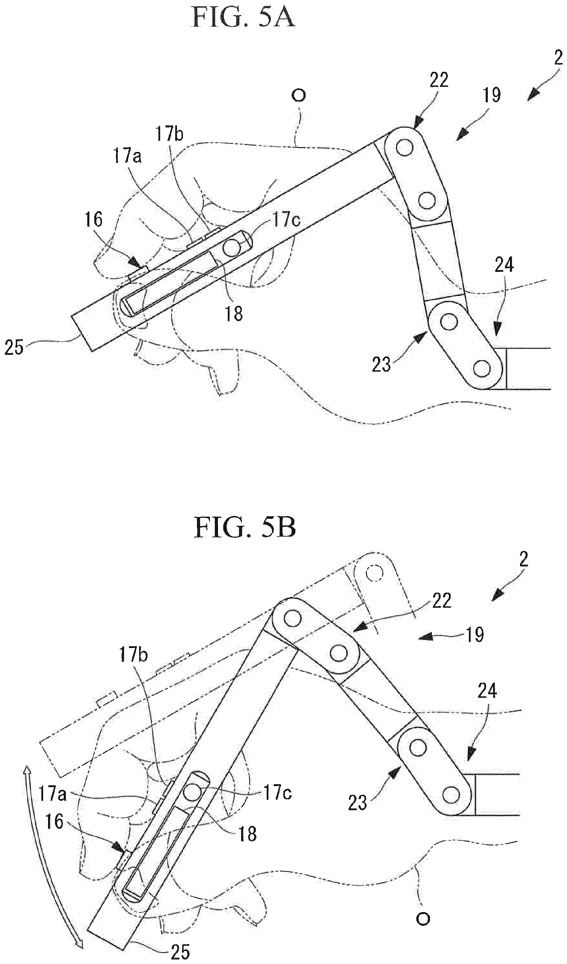

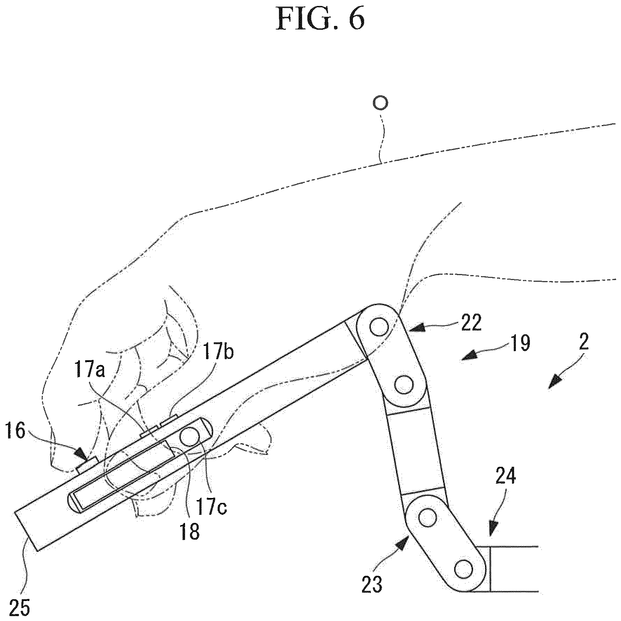

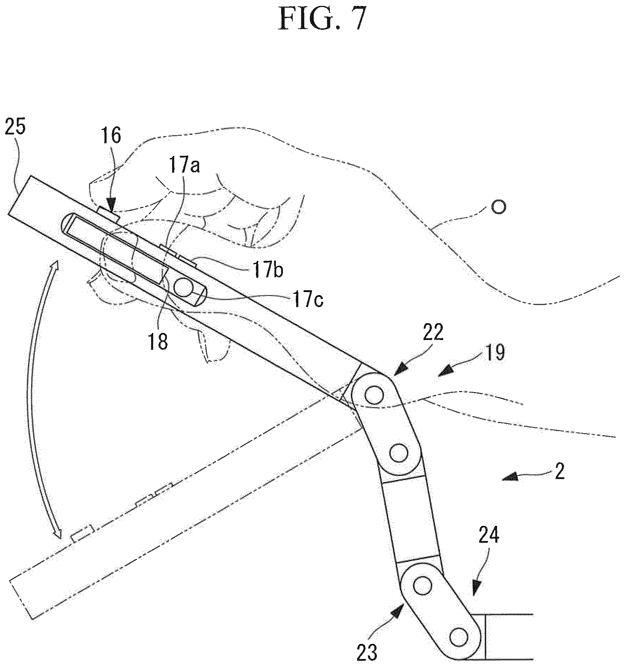

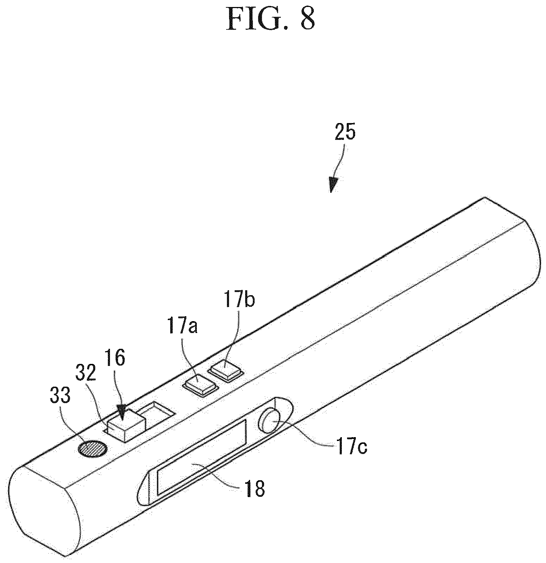

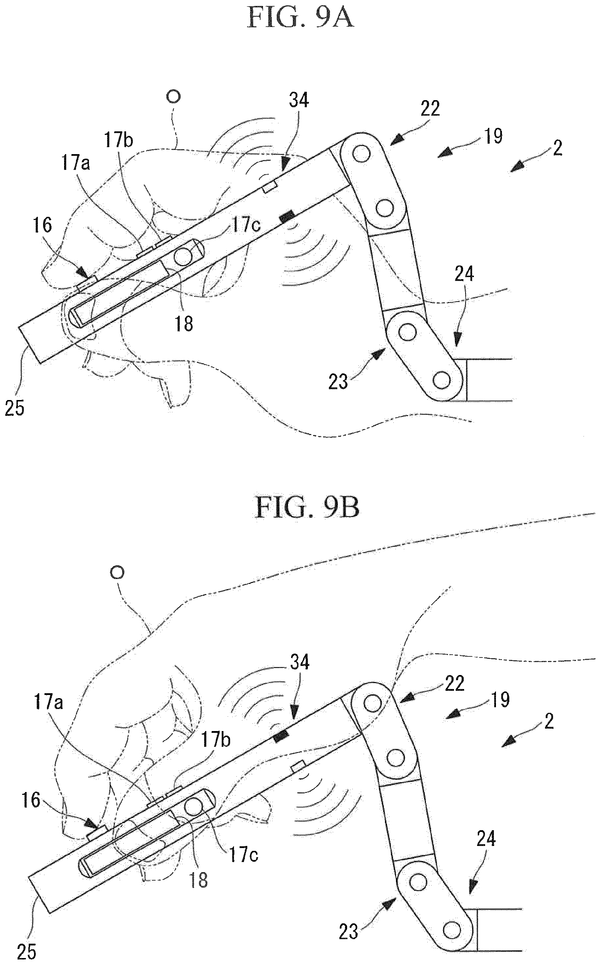

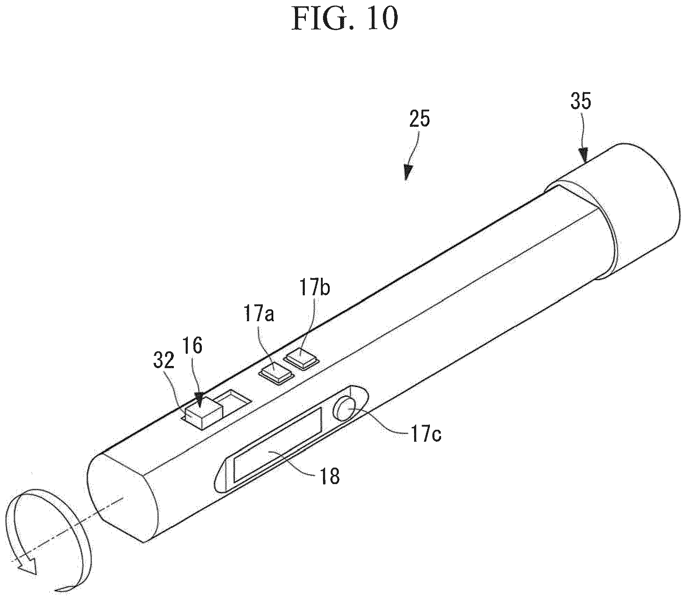

{Fig. 1} Fig. 1 illustrates the overall configuration of a medical manipulator system according to an embodiment of the present invention.
{Fig. 2} Fig. 2 illustrates a medical manipulator, an operation input device, and a controller used in the medical manipulator system in Fig. 1.
{Fig. 3} Fig. 3 is a perspective view illustrating an example of a grip section of the operation input device according to an embodiment of the present invention provided in the medical manipulator system in Fig. 1.
{Fig. 4A} Fig. 4A illustrates a state where the grip section in Fig. 3 is held like a pen, and a slide switch is operated with an index finger.
{Fig. 4B} Fig. 4B illustrates a state where the grip section in Fig. 3 is held like a pen, and a push button is operated with the index finger.
{Fig. 5A} Fig. 5A illustrates a substantially-upper-limit operational position of the grip section in Fig. 3 when it is held like a pen.
{Fig. 5B} Fig. 5B illustrates a lower-limit operational position of the grip section in Fig. 3 when it is held like a pen.
{Fig. 6} Fig. 6 illustrates a state where the method of holding the grip section is changed from the state in Fig. 5A such that the hand is placed over the grip section.
{Fig. 7} Fig. 7 illustrates a state where the grip section is swiveled further upward from the state in Fig. 6.
{Fig. 8} Fig. 8 is a perspective view illustrating a modification of the grip section in Fig. 3.
{Fig. 9A} Fig. 9A illustrates another modification of the grip section in Fig. 3 and shows a state where a proximity sensor is detecting the hand holding the grip section like a pen.
{Fig. 9B} Fig. 9B illustrates another modification of the grip section in Fig. 3 and shows a state where the proximity sensor is detecting the hand placed over the grip section to hold it.
{Fig. 10} Fig. 10 is a perspective view illustrating another modification of the grip section in Fig. 3.

### {Description of Embodiments}

An operation input device 2 and a medical manipulator system 1 according to an embodiment of the present invention will be described below with reference to the drawings.

As shown in Fig. 1, the medical manipulator system 1 according to this embodiment includes an operation input device 2 that is operated by an operator 0, a medical manipulator (manipulator) 3 to be inserted into the body cavity of a patient P, a controller 4 that controls the medical manipulator 3 based on an operation performed on the operation input device 2, and a monitor 5.

For example, as shown in Fig. 2, the medical manipulator 3 includes insertion sections 6 to be inserted into the body cavity of the patient P and distal-end moving units 7 to be inserted into the body of the patient P via channels R extending longitudinally through the insertion sections 6. Each distal-end moving unit 7 includes a long section 8 disposed within the channel R in a longitudinally movable manner, a movable section 9 provided at the distal end of the long section 8, a high-frequency gripper (end effector) 11 provided at the distal end of the movable section 9, and a distal-end driver 10 that is disposed at the proximal end of the long section 8 and that drives the movable section 9 by means of a power transmission member, such as a wire (not shown). The distal-end driver 10 includes an electrical driving source (not shown), such as a motor, for applying tension to the wire in accordance with an operation command from the controller 4.

As indicated by voided arrows in Fig. 2, each movable section 9 has two joints 12 and 13 that swivel the high-frequency gripper 11, disposed at the distal end, about two parallel axes, and a single joint 14 that rotates the high-frequency gripper 11 about the longitudinal axis of the long section 8. By moving these three joints 12, 13, and 14, each movable section 9 is capable of three-dimensionally positioning the distal end of each high-frequency gripper 11.

The medical manipulator 3 further includes two proximal-end moving units 15 that are connected to the proximal ends of the respective distal-end moving units 7 and that cause the distal-end moving units 7 to advance and retract in the longitudinal direction of the insertion sections 6. Each proximal-end moving unit 15 causes the corresponding long section 8 to bend in a direction orthogonal to the longitudinal direction thereof near the proximal end of the corresponding insertion section 6.

As shown in Fig. 2, the operation input device 2 according to this embodiment includes first operation units (arm units) 19 to be manually operated by the operator 0, a second operation unit (main device unit) 20 to be operated by the operator 0 using his/her wrist or arm, and a command transmission unit 21 that transmits manipulation commands input via these operation units 19 and 20 to the medical manipulator 3.

The first operation units 19 have a shape analogous to the movable sections 9 of the medical manipulator 3 and each have a grip section 25 supported by joints 22, 23, and 24 equal in number to the number of joints in each movable section 9. The operator 0 manually holds the grip section 25, so that each of the first operation units 19 is manually moved by the operator 0. Each of the first operation units 19 includes angle sensors (not shown) that detect the angles of the joints 22, 23, and 24 constituting the first operation unit 19.

The angle sensors generate electric signals according to the angles of the joints 22, 23, and 24. Thus, each of the first operation units 19 can receive a manipulation command from the hand of the operator 0 and can generate an operation command constituted of an electric signal.

As shown in Figs. 2 and 3, each grip section 25 has the shape of a rod that can be held by the operator 0 and the outer peripheral surface of which is provided with a slide sensor (operating section) 16, three push buttons 17a, 17b, and 17c, and a contact sensor (sensor) 18.

The slide sensor 16 is slidable along the longitudinal axis of the rod-shaped grip section 25 and can be used for adjusting the open and close angles of a gripper, which is one of the main functions of the high-frequency gripper 11 serving as an end effector. The slide sensor 16 includes a slider 32, which is slidable, and maintains the slider 32 at a release position of the index finger.

The two push buttons 17a and 17b of the three push buttons 17a, 17b, and 17c are arranged side-by-side in the sliding direction of the slide sensor 16. These push buttons 17a and 17b are on-off switches for applying high frequency electricity from the gripper, which is another one of the main functions of the high-frequency gripper 11 serving as an end effector. For example, the two push buttons 17a and 17b are used by being selectively pressed when performing medical treatment in an incision mode or a coagulation mode.

The contact sensor 18 and the remaining push button 17c are arranged along the longitudinal axis of the grip section 25 at positions 90° away from, for example, the slide sensor 16 in the circumferential direction of the grip section 25. The contact sensor 18 is disposed at a position where a finger comes into contact therewith when the grip section 25 is held, and can detect that the grip section 25 is in a held state. The push button 17c is, for example, a clutch button and can switch between connected and disconnected states of the linkage between the operation input device 2 and the medical manipulator 3 every time the push button 17c is pressed.

As shown in Figs. 4A and 4B, in this embodiment, the grip section 25 can be held like a pen by the operator 0. In this case, as shown in Fig. 4A, the operator 0 holds the grip section 25 by using his/her thumb and middle finger (not shown) to pinch it from opposite sides in the horizontal direction, so that the grip section 25 pinched between the two fingers is maintained in a stable orientation.

In this case, the index finger is not used for stably holding the grip section 25 but can be moved relatively freely without affecting the orientation of the grip section 25.

Accordingly, by disposing the contact sensor 18 at the position where the thumb is to be set when the grip section 25 is held in this manner and by disposing the slide sensor 16 at the position where the index finger is to be set, the thumb would come into contact with the contact sensor 18 so that a held state can be detected so long as the grip section 25 is held like a pen. In this case, as shown in Fig. 4A, the slide sensor 16 can be freely slid in the longitudinal direction of the grip section 25 by using the index finger, which is in a free state. Moreover, as shown in Fig. 4B, the two push buttons 17a and 17b arranged beside the slide sensor 16 can be pressed by using the index finger.

Furthermore, in a state where the grip section 25 is supported by the index finger and the middle finger, the thumb can be moved relatively freely. Thus, by moving the thumb to the position of the push button 17c from the state where the thumb is holding the grip section 25 and is in contact with the contact sensor 18, it is possible to switch between connected and disconnected states of the linkage between the operation input device 2 and the medical manipulator 3.

Furthermore, in this embodiment, from the state where the grip section 25 is held like a pen by setting the palm therebelow, as shown in Fig. 4A, the method of holding the grip section 25 can be changed to a state where the palm is placed over the upper surface of the grip section 25, as shown in Fig. 6.

In this case, if the holding method as in Fig. 6 is selected, the grip section 25 is held horizontally with the thumb and the middle finger, and the index finger is placed on the upper surface of the grip section 25 so as to be movable relatively freely.

The second operation unit 20 includes an arm table 26 fixed to the base of each of the first operation units 19 and a rectilinear mechanism 27 that supports the arm table 26 and each of the first operation units 19 in an integrally movable manner. The arm table 26 is disposed at a position where the operator 0 places his/her arm, particularly, near the wrist of the hand holding the grip section 25, when holding the grip section 25 of each of the first operation units 19.

The rectilinear mechanism 27 includes a slider 28 to which the arm table 26 and each of the first operation units 19 are fixed, and also includes a linear guide 29 that supports the slider 28 in a movable manner in two horizontal directions that are orthogonal to each other, as indicated by black arrows in Fig. 2. By moving the slider 28 horizontally by means of the arm placed on the arm table 26, each of the first operation units 19 can be positionally moved while maintaining the orientation in which each of the first operation units 19 is held. Accordingly, the second operation unit 20 can receive a manipulation command from the wrist or the arm of the operator 0 and can generate the force received from the wrist or the arm as two mechanical driving forces of the slider 28.

The command transmission unit 21 includes an electric-signal transmission unit 30 that connects the first operation units 19 and the distal-end drivers 10, and also includes a mechanical-power transmission unit 31 that connects the second operation unit 20 and the proximal-end moving units 15.

The electric-signal transmission unit 30 transmits an operation command constituted of an electric signal generated by each first operation unit 19 to the controller 4, and supplies a command signal generated by the controller 4 to motors of each distal-end driver 10. Based on the operation command generated by each of the first operation units 19, the controller 4 calculates the rotational amounts and the rotational velocities of the motors of each distal-end driver 10 and controls the motors.

Furthermore, the controller 4 controls each end effector 11 so as to change the open and close angles of the high-frequency gripper 11 in accordance with the slide position of the slider 32 of the slide sensor 16 provided in the grip section 25 of each of the first operation units 19. Moreover, when the push buttons 17a, 17b, and 17c are pressed, the controller 4 controls the functions allocated to the push buttons 17a, 17b, and 17c, that is, the power on/off states in the incision mode, the power on/off states in the coagulation mode, and the interruption of the clutch.

Furthermore, in the state where the grip section 25 is held, the controller 4 allows the operation input device 2 to control the medical manipulator 3 when the thumb is brought into contact with the contact sensor 18. When the thumb is moved away from the contact sensor 18, the controller 4 performs control to stop the medical manipulator 3.

The operation of the operation input device 2 and the medical manipulator system 1 according to this embodiment having the above-described configuration will be described below.

In order to medically treat an affected site inside the body of the patient P by using the medical manipulator system 1 according to this embodiment, the insertion section 6 of the medical manipulator 3 is inserted into the body cavity of the patient P, and the movable section 9 and the long section 8 are inserted into the body of the patient P via the channel R in the insertion section 6.

Then, in a state where the movable section 9 is disposed adjacent to the affected site in the body cavity, the operator O operates the operation input device 2 while viewing the monitor 5 to check an image acquired by an endoscope (not shown). In order to operate the operation input device 2, the operator 0 holds the grip section 25 of each of the first operation units 19 with one hand and places the arm of that hand on the arm table 26 of the second operation unit 20.

Then, when the operator 0 applies force from the arm to the arm table 26, the slider 28 to which the arm table 26 is fixed moves in the direction of that force. The amount of movement of the slider 28 is split into a rectilinear movement amount in the forward-rearward direction and a rectilinear movement amount in the left-right direction, so that the distal-end position of the high-frequency gripper 11 located at the distal end of the movable section 9 can be manually moved roughly in the forward-rearward direction and the left-right direction.

Meanwhile, when the grip section 25 of each of the first operation units 19 manually held by the operator 0 is moved, the amount of movement thereof is detected by the angle sensors provided at the joints 22, 23, and 24 and is transmitted to the controller 4 as an electric signal. The controller 4 calculates electrical operation commands for moving the joints 12, 13, and 14 of the movable section 9 such that they have angles identical to the angles of the joints 22, 23, and 24 detected by the angle sensors, and supplies the operation commands to the motors connected to the joints 12, 13, and 14. Accordingly, the distal-end position of the high-frequency gripper 11 provided at the distal end of the movable section 9 is electrically moved precisely as designated by the movement of the hand.

In this case, as shown in Figs. 4A and 4B, the operator 0 is holding the grip section 25 like a pen and can stably hold the grip section 25 by pinching the grip section 25 with his/her thumb and middle finger from opposite sides in the horizontal direction. Because the thumb is in contact with the contact sensor 18 provided in the grip section 25, the controller 4 moves the medical manipulator 3 based on the angular information of the joints 22, 23, and 24 from the angle sensors in accordance with the position and the orientation of the grip section 25.

Then, when the operator 0 slides the slider 32 of the slide sensor 16 so as to grip biological tissue with the high-frequency gripper 11, the controller 4 opens and closes the high-frequency gripper 11 in accordance with the position of the slider 32. In this case, since the index finger of the hand of the operator 0 holding the grip section 25 is set at a position where the index finger is in contact with the slider 32, the slider 32 can be readily slid in the longitudinal direction of the grip section 25 by moving the index finger.

Specifically, because the slider 32 is operated using the index finger, which is movable relatively freely, relative to the grip section 25 stably held by being pinched with the thumb and the middle finger, the grip section 25 does not wobble when the slider 32 is being operated, so that the high-frequency gripper 11 can be opened and closed without the distal-end position of the high-frequency gripper 11 of the medical manipulator 3 fluctuating.

In particular, because the slide sensor 16 used is of a type in which the slider 32 is slid in the longitudinal direction of the grip section 25, the force applied from the index finger to the slider 32 acts in the longitudinal direction of the grip section 25. In other words, since force that presses the grip section 25 does not act in the direction intersecting the longitudinal axis thereof, the effect of preventing wobbling of the grip section 25 is enhanced.

Then, by pressing the push button 17a with the index finger in the state where biological tissue is gripped by means of the high-frequency gripper 11, high frequency electricity is applied to the biological tissue from the high-frequency gripper 11, whereby medical treatment, such as incision or coagulation, can be performed on the biological tissue. Because the biological tissue is gripped by the high-frequency gripper 11 during the medical treatment, there is no problem even if the grip section 25 wobbles more or less due to pressing of the push button 17a or 17b.

When the linkage between the operation input device 2 and the medical manipulator 3 is disconnected, the operator 0 slides his/her thumb to press the push button 17c. Because the medical manipulator 3 stops as soon as the thumb is moved away from the contact sensor 18, there is no problem even if the grip section 25 wobbles more or less due to shifting of the thumb or pressing of the push button 17c. Moreover, because the push button 17c serving as a clutch button is disposed away from the position of the thumb when the grip section 25 is being held, the clutch button can be prevented from being erroneously operated, and the clutch can also function to interrupt the operation.

In order to move the grip section 25 in a state where the grip section 25 is held like a pen, for example, if the grip section 25 is to be moved vertically, as shown in Figs. 5A and 5B, the movement range of the grip section 25 is limited to the range within which the distal end of the grip section 25 is oriented downward, as shown in Figs. 5A and 5B, due to the limited movement range of the wrist. In particular, the wrist cannot be oriented further upward from the state shown in Fig. 5A.

In this embodiment, the method of holding the grip section 25 is changed by using the same hand, as shown in Fig. 6, so that the distal end of the grip section 25 can be moved to an upward-oriented angular position, as shown in Fig. 7.

In this case, even after the holding method is changed, the grip section 25 can be stably held by being pinched with the thumb and the middle finger of the same hand from opposite sides in the horizontal direction, and the slide sensor 16 and the push buttons 17a and 17b can be pressed using the index finger, which can be moved relatively freely.

Accordingly, the operation input device 2 and the medical manipulator system 1 according to this embodiment are advantageous in that the operation for opening and closing the gripper, which is one of the main functions of the high-frequency gripper 11 serving as an end effector, can be performed while keeping the orientation of the end effector 11 stable, and in that limitations in the manipulation amount due to the movable range of the wrist can be alleviated.

The slide sensor 16 used in this embodiment is of a type in which the slide position of the slider 32 is maintained. Alternatively, the slide sensor 16 may be biased by a spring in a direction in which the high-frequency gripper 11 opens. With regard to movement in the direction in which the high-frequency gripper 11 is closed by an unlockable latchet mechanism, the slider 32 may be lockable at each position. Accordingly, the index finger can be moved away from the slider 32 while maintaining the state where the biological tissue is gripped by the high-frequency gripper 11, and the same index finger can be used to press the push button 17a for applying electricity.

Furthermore, as an alternative to the slide sensor 16 described above, which is of a type used by physically sliding the slider 32, a plurality of contact sensors 18 may be arranged in the longitudinal direction on the surface of the grip section 25, and the slide position may be determined in accordance with an output from each contact sensor 18 that detects contact with the index finger.

Furthermore, as an alternative to this embodiment in which the high-frequency gripper 11 is used as an end effector, a gripper that does not perform high frequency output or an electric scalpel that only performs high frequency output may be used. In the case of the gripper alone, the slide sensor 16 may be the only component to be operated with the index finger.

In the case of the electric scalpel, the slide sensor 16 may be used to switch between on and off states for applying electricity, which is the main function of the electric scalpel. In this case, the two push buttons 17a and 17b may be used for switching between the incision mode and the coagulation mode, and a display lamp 33 that displays the selected mode by means of color may be disposed in the grip section 25, as shown in Fig. 8. As an alternative to the two push buttons 17a and 17b, a single switch may be provided for switching between the modes. A treatment tool other than a gripper or an electric scalpel may be used as the end effector 11.

Furthermore, as shown in Figs. 9A and 9B, a proximity sensor (position detector) 34 that is disposed on the upper and lower surfaces of the grip section 25 and that detects whether a hand of the operator 0 is set at the upper side or the lower side of the grip section 25 may be used in place of the contact sensor 18 or in addition to the contact sensor 18.

Accordingly, it is possible to determine whether the grip section 25 is held like a pen, as shown in Fig. 9A, or whether the grip section 25 is held by placing the hand over it, as shown in Fig. 9B.

As described above, because the movement range of the grip section 25 varies depending on how it is held, a software limiter that limits the movement of the medical manipulator 3 may be switched in accordance with the detected holding method. Moreover, the functions associated with the slide sensor 16 and the push buttons 17a and 17b may be switched in accordance with how the grip section 25 is held. Furthermore, the position of the slide sensor 16 may be designed structurally analogous to the position of the end effector 11, and calculated reference coordinates may be switched in accordance with how the grip section 25 is held.

Furthermore, because the joint 24 is provided for rotating the entirety of each of the first operation units 19, when the joint 24 is rotated by operating the grip section 25, the grip section 25 is changed in orientation so as to be twisted about the longitudinal axis thereof. When the grip section 25 is rotated about its longitudinal axis, it is conceivable that it is difficult to hold the grip section 25 since the positions of the fingers holding the grip section 25 also rotate.

As shown in Fig. 10, a rotational supporter 35 that supports the grip section 25 in a rotatable manner about the longitudinal axis thereof may be provided. The rotation of the grip section 25 by the rotational supporter 35 does not affect the movement of the medical manipulator 3. Accordingly, the grip section 25 can be held and operated such that, for example, the finger operating, for example, the slide sensor 16 is set thereon comfortably.

In the above-described embodiment, the medical manipulator 3 stops moving as soon as the thumb is moved away from the contact sensor 18. Alternatively, the medical manipulator 3 may be stopped when a predetermined time period elapses after the thumb is moved away from the contact sensor 18. In this case, a time period necessary for changing the method of holding the grip section 25 may be set as the predetermined time period.

Accordingly, the medical manipulator 3 can be continuously manipulated without interruption when changing the method of holding the grip section 25. Moreover, a drop detection sensor, such as an acceleration sensor, may be provided in addition to the contact sensor 18. In a case where it is detected that the grip section 25 has been dropped, the medical manipulator 3 may be stopped even within the predetermined time period.

Although the contact sensor 18 described above is of a type that is disposed at the position of the thumb, the contact sensor 18 used may alternatively be of a type that is disposed toward the middle finger.

Furthermore, the contact sensor 18 may be provided in the form of a ring in the circumferential direction of the grip section 25. Accordingly, any of the fingers can be brought into contact with the contact sensor 18 when changing the method of holding the grip section 25.

Furthermore, in this embodiment, a type detector (not shown) that detects the type of medical manipulator 3 may be provided.

Then, the controller 4 changes the correspondence relationship between the slide sensor 16 and the main function of the end effector 11 in accordance with the type of medical manipulator 3 detected by the type detector, so that the medical manipulator 3 can be manipulated using the same operation input device 2 even if the medical manipulator 3 is replaced with another one.

### {Reference Signs List}

- 1: medical manipulator system
- 2: operation input device
- 3: medical manipulator (manipulator)
- 4: controller
- 11: high-frequency gripper (end effector)
- 16: slide sensor (operating section)
- 18: contact sensor (sensor)
- 19: first operation unit (arm unit)
- 20: second operation unit (main device unit)
- 25: grip section
- 34: proximity sensor (position detector)
- 35: rotational supporter
- O: operator

## Claims

1. An operation input device for inputting a manipulation command to a manipulator having an end effector for observing or medically treating an affected site, the operation input device comprising:
a grip section that is holdable by changing a method of holding the grip section with the same hand of an operator; and
an operating section that is provided in the grip section and that is operated with a single finger, which is the same before and after the holding method is changed, so as to manipulate a main function of the end effector.

2. The operation input device according to Claim 1, further comprising a sensor that is provided in the grip section and that detects a holding state of a hand of the operator holding the grip section.

3. The operation input device according to Claim 2, wherein the sensor is a contact sensor.

4. The operation input device according to Claim 2 or 3,
wherein the sensor detects a holding-method changing operation performed on the grip section by the operator.

5. The operation input device according to Claim 1, wherein the operating section is a slide sensor that adjusts an amount by which the main function of the end effector is manipulated when the single finger is slid relative to the grip section.

6. The operation input device according to any one of Claims 1 to 5, further comprising a main device unit and an arm unit that connects the grip section to the main device unit in a movable manner,
wherein the arm unit includes a rotational supporter that supports the grip section in a rotatable manner about a longitudinal axis thereof.

7. A medical manipulator system comprising:
a manipulator having an end effector for observing or medically treating an affected site;
the operation input device according to any one of Claims 1 to 6; and
a controller that controls the manipulator based on a manipulation command input via the operation input device.

8. The medical manipulator system according to Claim 7,
wherein the operation input device includes a position detector that detects a position of the hand of the operator relative to the grip section, and
wherein the controller changes a command signal for the manipulator in accordance with the position of the hand of the operator detected by the position detector.

9. The medical manipulator system according to Claim 8,
wherein the operation input device includes a plurality of the operating sections, and
wherein the controller changes a correspondence relationship between each operating section and the main function of the end effector in accordance with the position of the hand of the operator detected by the position detector.

10. The medical manipulator system according to any one of Claims 7 to 9, further comprising:
a type detector that detects a type of the manipulator,
wherein the controller changes a correspondence relationship between the operating section and the main function of the end effector in accordance with the type of the manipulator detected by the type detector.

11. A medical manipulator system comprising:
a manipulator having an end effector for observing or medically treating an affected site;
the operation input device according to any one of Claims 2 to 4; and
a controller that controls the manipulator based on a manipulation command input via the operation input device,
wherein, in a case where the sensor detects that the grip section is not held by the hand of the operator, the controller performs control so as to stop the manipulator from moving.
